# EUROPEAN PATENT APPLICATION

(11) **EP 3 970 727 A1**
(43) Date of publication of application: **23.03.2022**
(21) Application number: 20196498.8
(22) Date of filing: 16.09.2020
(51) Int. Cl.: A61K 31/635, A61K 31/00, A61K 38/20, A61P 29/00, A61P 35/00, A61K 41/00, A61N 5/10, G01N 33/50

(54) **MEANS FOR REDUCING RADIOTHERAPY RESISTANCE AND ADVERSE EFFECTS**

(71) Applicant: Johann Wolfgang Goethe-Universität Frankfurt, 60323 Frankfurt am Main (DE); Chemotherapeutisches Forschungsinstitut Georg-Speyer-Haus, 60596 Frankfurt am Main (DE)
(72) Inventor: Greten, Florian, 61352 Bad Homburg (DE); Nicolas, Adele, 65451 Kelsterbach (DE); Rödel, Claus, 60590 Frankfurt am Main (DE); Fokas, Emmanouil, 60590 Frankfurt am Main (DE)
(74) Representative: Kuttenkeuler, David

(57) **Abstract**

The invention is based on the modulation of cancer-associated fibroblasts (CAFs) in the treatment of proliferative disorders by radiotherapy. By reducing CAF sensitivity to cellular senescence adverse immune reactions upon radiotherapy of solid tumours and subsequent treatment resistance could be avoided. The invention pertains to senolytics or Interleukin 1 (ILi) signalling inhibitors for use in a method of treating solid tumours and in the treatment or prevention of inflammatory adverse effects upon radiation therapy in context of a cancer treatment.

## Description

### FIELD OF THE INVENTION

The invention is based on the modulation of cancer-associated fibroblasts (CAFs) in the treatment of proliferative disorders by radiotherapy. By reducing CAF sensitivity to cellular senescence adverse immune reactions upon radiotherapy of solid tumours and subsequent treatment resistance could be avoided. The invention pertains to senolytics or Interleukin 1 (IL1) signalling inhibitors for use in a method of treating solid tumours and in the treatment or prevention of inflammatory adverse effects upon radiation therapy in context of a cancer treatment.

### DESCRIPTION

The composition and polarization of the cellular constituents of the tumor microenvironment play an important role in tumorigenesis, yet they are being increasingly appreciated as attractive targets for cancer therapy in combination with standard cytotoxic treatments (1). Rectal cancer therapy represents a particularly good example for the successful implementation of a multimodal approach in cancer treatment (2). The establishment of neoadjuvant therapy concepts based on chemoradiotherapy (CRT) or short-course radiotherapy prior to surgical resection has been a turning point in the treatment of rectal cancer leading to substantially reduced local recurrence rates and, partly, improved survival (3, 4). Despite identical tumor histology, comparable tumor stages and uniform therapy individual patient response to neoadjuvant therapy can range from a clinically and pathologically complete remission to progression under treatment (5). This individually variable tumor response is routinely assessed by ypTNM (i.e. tumor stage after neoadjuvant treatment) where pathological complete response (pCR i.e. complete tumor regression and lack of residual lymph nodes: ypToNoMo) after CRT has a strong prognostic role and correlates with significantly better DFS and OS compared to patients without pCR (non-pCR) (5-7). However, a comprehensive understanding of the molecular basis that defines and predicts individual therapy response is lacking.

Thus, it is an objection of the invention to provide a solution for harmful radiotherapy side effects and the development of tumour resistances to radiotherapy.

### BRIEF DESCRIPTION OF THE INVENTION

Generally, and by way of brief description, the main aspects of the present invention can be described as follows:
In **the first aspect**, the invention pertains to a compound for use in the treatment of a solid tumour in a subject, wherein the subject is treated by reducing sensitivity of cancer-associated fibroblasts (CAFs) to cellular senescence and concomitant or subsequent irradiation therapy targeting the solid tumour in the subject.

In **the second aspect**, the invention pertains to a compound for use in treating inflammatory side effects of irradiation therapy in a subject suffering from a solid tumour disease, wherein the compound is a senolytic or IL-1/IL-1R inhibitor.

In **the third aspect**, the a method for identifying or characterizing a compound suitable for increasing sensitivity of a solid tumour disease to irradiation therapy, the method comprising the steps of
- Contacting a candidate compound and a cancer-associated fibroblast (CAF),
- Inducing a p53 senescence program in the CAF contacted with the candidate compound,
wherein a reduced pro-inflammatory phenotype in the a contacted with the candidate compound compared to a CAF not contacted with the candidate compound indicates the candidate compound suitable for increasing sensitivity of a solid tumour disease to irradiation therapy.

In **the fourth aspect**, the invention pertains to a method of treating a solid tumour disease in a subject in need thereof, the method comprising the steps of administering to the subject a therapeutically effective amount of a compound recited in any of the preceding claims, and irradiating the solid tumour in the subject.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **the first aspect**, the invention pertains to a compound for use in the treatment of a solid tumour in a subject, wherein the subject is treated by reducing sensitivity of cancer-associated fibroblasts (CAFs) to cellular senescence and concomitant or subsequent irradiation therapy (radiotherapy) targeting the solid tumour in the subject.

As used herein, the term "fibroblast" may refer to a cell of the connective tissue of a mammal, which constitutes components of the fibrous connective tissue. As used herein, the term "cancer-associated fibroblast (CAF)" refers to a kind of fibroblast associated or located in the direct tumour environment, such as a fibroblast located directly adjacent to or within a solid tumour. Generally CAF cells exist in the tumour stroma, and are known to be involved in the tumour growth, formation of tumour blood vessels, and invasion and metastasis of tumour cells in most cancers. CAFs are CD45"EpCAM"CD3 CD29+ stromal cells. CAFs are one of the most abundant stromal components with morphology similar to that of myofibroblasts.

As used herein, the term "treatment" or "treat" refer to both prophylactic or preventive treatment as well as curative or disease modifying treatment, including treatment of patients at risk of contracting the disease or suspected to have contracted the disease as well as patients who are ill or have been diagnosed as suffering from a disease or medical condition, and includes suppression of clinical relapse. The treatment may be administered to a patient having a medical disorder or who ultimately may acquire the disorder, in order to prevent, cure, delay the onset of, reduce the severity of, or ameliorate one or more symptoms of a disorder or recurring disorder, or in order to prolong the survival of a patient beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a patient during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a patient during treatment of an illness, e.g., to keep the patient in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., disease manifestation, etc.]).

The terms "irradiation therapy" or "radiotherapy" have their general meaning in the art and refer to photon (X) radiotherapy and proton (P) radiotherapy. The term "irradiation therapy" or "radiotherapy" also refers to photon irradiation and proton irradiation. The term "irradiation therapy" or "radiotherapy" also refers to single irradiation (SI) and multiple irradiations (MI) such as described in the example. The term "irradiation therapy" or "radiotherapy" also refers to radiation therapy using radio therapeutic agent administered to the patient afflicted with solid cancer.

Irradiation therapy or radiotherapy is the medical use of irradiation (i.e. ionizing radiation) as part of cancer treatment to control malignant cells. Ionizing radiation deposits energy that injures or destroys cells in the area being treated (the target tissue) by damaging their genetic material, making it impossible for these cells to continue to grow. One type of radiation therapy commonly used involves photons, e.g. X-rays. Depending on the amount of energy they possess, the rays can be used to destroy cancer cells on the surface of or deeper in the body. The higher the energy of the x-ray beam, the deeper the x-rays can go into the target tissue. Linear accelerators and betatrons produce x-rays of increasingly greater energy. The use of machines to focus radiation (such as x-rays) on a cancer site is called external beam radiotherapy. Gamma rays are another form of photons used in radiotherapy. Gamma rays are produced spontaneously as certain elements (such as radium, uranium, and cobalt 60) release radiation as they decompose, or decay. Another technique for delivering radiation to cancer cells is to place radioactive implants directly in a tumour or body cavity. This is called internal radiotherapy. Brachytherapy, interstitial irradiation, and intracavitary irradiation are types of internal radiotherapy. In this treatment, the radiation dose is concentrated in a small area. A further technique is intra-operative irradiation, in which a large dose of external radiation is directed at the tumour and surrounding tissue during surgery.

Another approach is particle beam radiation therapy. This type of therapy differs from photon radiotherapy in that it involves the use of fast-moving subatomic particles to treat localized cancers. Some particles (protons, neutrons, pions, and heavy ions) deposit more energy along the path they take through tissue than do x-rays or gamma rays, thus causing more damage to the cells they hit. This type of radiation is often referred to as high linear energy transfer (high LET) radiation. Radio-sensitizers make the tumour cells more likely to be damaged, and radio -protectors protect normal tissues from the effects of radiation.

A person of ordinary skill in the radiotherapy art knows how to determine an appropriate dosing and application schedule, depending on the nature of the disease and the constitution of the patient. In particular, the person knows how to assess dose-limiting toxicity (DLT) and how to determine the maximum tolerated dose (MTD) accordingly. More particularly, the amount of radiation used in photon radiation therapy is measured in Gray (Gy), and varies depending on the type and stage of cancer being treated. Many other factors are considered by radiation oncologists when selecting a dose, including whether the patient is receiving chemotherapy, patient co -morbidities, whether radiation therapy is being administered before or after surgery, and the degree of success of surgery. Moreover, the total dose is often fractionated. Fractionation regimes are individualized between different radiotherapy centers. The typical fractionation schedule for adults is 1 to 2 Gy per day, five days a week. In some cases, two fractions per day are used near the end of a course of treatment. This schedule is known as a concomitant boost regimen or hyperfractionation. Thus, in another preferred embodiment, the radiotherapy can be applied at a dose in a range from about 1 to 80Gy, about 10 to 55Gy, preferably from about 15 to 50 Gy, such as 20 to 40Gy, concretely from about 20 to 35 Gy, and more concretely from about 25 to 30 Gy.

In specifically preferred embodiments of the invention, of the solid tumour is a rectal tumour, the fractionation schedule for adults is about 1,5 to about 2, preferably about 1,8 to about 2 Gy daily, which can be increased in some cases to about 50-50,4 Gy, in some cases about 54 Gy. As an alternative schedule in this embodiment, the schedule is about 5 times about 5 Gy up to about 25 Gy

The term "cellular senescence" refers to stable cell cycle arrest accompanied by a set of characteristic morphological and physiological features that distinguish senescent cells from proliferating cells, as well as arrested quiescent or terminally differentiated cells (Kosar et al. Cell Cycle 2011 Feb 1;10(3):457-68. doi: 10.4161/cc.10.3.14707).

Preferably, a cellular senescence of a CAF is a p53 dependent cellular senescence, hence, includes a cellular senescence program that involves the p53 protein.

A "reduced" sensitivity is observed if a CAF treated according to the invention compared to a CAF not treated according to the invention has a lower likelihood to enter cellular senescence. In order to determine such reduction any of the methods of the attached examples for testing cellular senescence or the occurrence of the harmful inflammatory phenotype may be used.

The present invention is predicated upon an observed induction of cellular senescence in CAF upon tumour irradiation during cancer therapy. As shown in the attached examples, CAFs are polarized towards an inflammatory phenotype triggering cellular senescence upon irradiation which results in irradiation resistance. Therefore, the invention intends to reduce CAF sensitivity to cellular senescence during irradiation therapy and thereby to reduce or avoid radiotherapy resistance.

As used herein, the term "subject" refers to any mammal, preferably a human, suffering from cancer. Yet, the method of the invention is particularly useful for treating a subject suffering from solid tumours, such as a glioma, a lymphoma, a melanoma, a sarcoma, a head and neck tumor, a breast or a lung cancer, or colon cancer, colorectal cancer, rectal cancer, cancer of the stomach. The term subject, as used herein, refers in certain preferred embodiments to any human or animal (such as a subject suffering from cancer) which undergoes a radiotherapy-based treatment. The term "subject" may be substituted with the term "patient" where appropriate.

In a preferred embodiment of the present invention, the cancer is a solid tumour. The term "solid tumour" especially means breast cancer, ovarian cancer, cancer of the colon and generally the G1 (gastro-intestinal) tract, cervix cancer, lung cancer, in particular small- cell lung cancer, and non- small-cell lung cancer, head and neck cancer, bladder cancer, cancer of the prostate or Kaposi's sarcoma. The present combination inhibits the growth of solid tumours, but also liquid tumours. Furthermore, depending on the tumour type and the particular combination used a decrease of the tumour volume can be obtained. The combinations disclosed herein are also suited to prevent the metastatic spread of tumours and the growth or development of micro-metastases. The combinations disclosed herein are in particular suitable for the treatment of poor prognosis patients, especially such poor prognosis patients having metastatic melanoma or pancreatic cancer.

Certain preferred solid tumours are such solid tumours treatable by radiotherapy and being of epithelial origin, such as pancreatic cancer, bladder cancer, prostate cancer, ovarian cancer, colorectal cancer (CRC), breast cancer, mesothelioma, renal cancer, lung cancer, hepatocellular cancer, cervical cancer, gastric cancer, esophageal cancer, head and neck cancer, melanoma, neuroendocrine cancer; preferably the solid tumour is rectal cancer.

However, any cancer that is in general treated by radiotherapy shall be comprised in certain preferred embodiments. In particular, the present invention relates to radiotherapy treatable cancer types selected from glioblastoma, lung cancer including small cell lung cancer and non-small cell lung cancer, testicular cancer, ovarian cancer, sarcoma, retinoblastoma, prostate cancer, osteosarcoma, neuroblastoma, multiple myeloma, non-hodgkin's lymphoma, hodgkin's lymphoma, acute myeloid leukemia, breast cancer, gastric cancer, brain tumors, melanoma, colorectal cancer (CRC), kidney cancer, such as e.g. renal cell carcinoma (RCC), liver cancer, acute myelogenous leukemia (AML), chronic myeloid leukemia (CML), acute lymphocytic leukemia.

Preferably a tumour selected for a treatment in accordance with the herein disclosed invention is a tumour that contains or is associated with CAFs.

A preferred tumour disease treatable in accordance with the invention is a tumour that already developed a resistance phenotype to radiotherapy. In this context it may be preferred that the subject treated in accordance with the invention has already received a first-line irradiation therapy, and has developed a resistance or reduced sensitivity to irradiation therapy.

The compound used as medicine in accordance with the present invention is selected from (i) an inhibitor of IL-1 signalling, or (ii) a senolytic compound. Such compound for use according to the invention has an activity towards CAFs selected from inhibition of Il-1 signalling in CAFs and/or induction of cellular senescence, preferably p53 driven, in CAFs, and/or is an inhibitor/antagonist of inflammatory CAF polarization, such as radiotherapy induced inflammatory CAF polarization.

In the present invention, the term "IL-1 inhibitor", or any grammatically equivalent term, refers to a compound which typically decreases or neutralizes a biological activity of IL-1 a (IL-1 alpha) and/or IL-1 b (IL-1 beta). This inhibitor is preferably a direct inhibitor of IL-1 a and/or IL-1 b, or of a receptor thereof. This means that the inhibitor typically directly decreases or neutralizes a biological activity of IL-1 or its receptor, or in other words, that the IL-1 decreased or neutralized biological activity is not the result of the action of an intermediate compound other than IL-1 receptor.

Examples of IL-1 inhibitors of interest are antibodies directed against IL-1 a and/or IL-1 b and/or IL-1 receptor, aptamers or spiegelmers directed against IL-1 a and/or IL-1 b and/or IL-1 receptor, inhibitory nucleic acid sequences directed against IL-1 a and/or IL-1 b and/or IL-1 receptor, IL-1 receptor antagonists, and small molecules directed against IL-1 a and/or IL- 1 b and/or IL-1 receptor. According to particular embodiments of the present invention, the IL-1 inhibitor specifically inhibits IL-1 a, specifically IL-1 b, specifically inhibits IL- 1 receptor, or specifically inhibits both IL-1 a and IL-1 b.

Inhibitors of IL-1 signalling, are in certain embodiments the following specific molecules and/or molecular classes: the IL-1 inhibitor may be one selected from a polypeptide, peptide, glycoprotein, a peptidomimetic, an antibody or antibody-like molecule (such as an intra-body); a nucleic acid such as a DNA or RNA, for example an antisense DNA or RNA, a ribozyme, an RNA or DNA aptamer, siRNA, shRNA and the like, including variants or derivatives thereof such as a peptide nucleic acid (PNA); a genetic construct for targeted gene editing, such as a CRISPR/Cas9 construct and/or guide RNA/DNA (gRNA/gDNA) and/or tracrRNA; a hetero-bifunctional compound (such as a PROTAC or a HyT molecule); a carbohydrate such as a polysaccharide or oligosaccharide and the like, including variants or derivatives thereof; a lipid such as a fatty acid and the like, including variants or derivatives thereof; or a small organic molecules including but not limited to small molecule ligands, or small cell-permeable molecules.

The IL-1 inhibitor of the invention may be in a prodrug form. Prodrugs forms of a particular compound are those other compounds (such as ester forms of the particular compound) that upon administration to a subject undergo chemical conversion under physiological conditions to provide the particular compound. Additionally, prodrugs can be converted to the particular compound by chemical or biochemical methods in an ex vivo environment. For example, prodrugs can be slowly converted to the particular compound when, for example, placed in a transdermal patch reservoir with a suitable enzyme or chemical reagent. Exemplary prodrugs are esters or amides which are hydrolysable *in vivo.*

Preferred inhibitors are for example selected from rilonacept, anakinra, or biosimilars thereof, or an IL-1 or IL-1R antibody (preferably antibody against IL-1α/IL-1RA), such antibody may be selected from anti-IL-1R1 antibody AMG 108 (Amgen), chimeric, Canakinumab, humanized or human antibody directed to IL-1 alpha or beta (such as CDP-484, Celltech) or the IL-1 receptor (for example, AMG-108, Amgen; R-1599, Roche).

In particular preferred are inhibitors of IL-1 a and/or IL-1 b for use according to the invention or comprised in the pharmaceutical composition for use according to the invention selected from the group consisting of canakinumab (Haris^{®}, a human monoclonal antibody of IgG1 /kappa isotype targeted at interleukin-i beta) and anakinra (Kineret^{®}, an interleukin-i receptor antagonist).

An alternative to the use of IL-1 signalling inhibitors is the use of senolytic agents or "senolytics". The term "senolytic" or "senolytic agent" in context of the invention shall refer both to agents that inhibit a cell entering the senescence program or alternatively (as the term suggest) selectively kill senescent cells. Preferably, the term "senolytic" refers to a small molecule that can selectively induce death of senescent cells. Senolytics may comprise at least one of the listed compounds: venetoclax, or quercetin, fisetin, luteolin, curcumin, A1331852, A1155463, geldanamycin, tanespimycin, alvespimycin, piperlongumine, FOXO₄ - related peptide, Nutlin3a, dasatinib, navitoclax, obatoclax, veliparib, ABT-737, ABT-199 and all the anti-cancer drugs acting as a PARP, Bcl-2, Bcl-XL and Bcl-w proteins inhibitor and thereof.

Typically the inhibitors according to the invention as described above are administered to the subject in a therapeutically effective amount.

By a "therapeutically effective amount" of the compound (IL-1 signalling inhibitor or senolytic) of the present invention as above described is meant a sufficient amount of the compound (IL-1 signalling inhibitor or senolytic) for treating solid cancer at a reasonable benefit/risk ratio applicable to any medical treatment. It will be understood, however, that the total daily usage of the inhibitors and compositions of the present invention will be decided by the attending physician within the scope of sound medical judgment. The specific therapeutically effective dose level for any particular patient will depend upon a variety of factors including the disorder being treated and the severity of the disorder; activity of the specific compound (IL-1 signalling inhibitor or senolytic) employed; the specific composition employed, the age, body weight, general health, sex and diet of the patient; the time of administration, route of administration, and rate of excretion of the specific compound (IL-1 signalling inhibitor or senolytic) employed; the duration of the treatment; drugs used in combination or co-incidental with the specific compound (IL-1 signalling inhibitor or senolytic) employed; and like factors well known in the medical arts. For example, it is well within the skill of the art to start doses of the compound (IL-1 signalling inhibitor or senolytic) at levels lower than those required to achieve the desired therapeutic effect and to gradually increase the dosage until the desired effect is achieved. However, the daily dosage of the products may be varied over a wide range from 0.01 to 1,000 mg per adult per day. Typically, the compositions contain 0.01, 0.05, 0.1, 0.5, 1.0, 2.5, 5.0, 10.0, 15.0, 25.0, 50.0, 100, 250 and 500 mg of the compound (IL-1 signalling inhibitor or senolytic) of the present invention for the symptomatic adjustment of the dosage to the patient to be treated. A medicament typically contains from about 0.01 mg to about 500 mg of the compound (IL-1 signalling inhibitor or senolytic) of the present invention, preferably from 1 mg to about 100 mg of the compound (IL-1 signalling inhibitor or senolytic) of the present invention. An effective amount of the drug is ordinarily supplied at a dosage level from 0.0002 mg/kg to about 20 mg/kg of body weight per day, especially from about 0.001 mg/kg to 7 mg/kg of body weight per day. The dose it preferably adjusted to reduce, overcome or avoid the occurance of radiotherapy resistance.

In a particular embodiment, the compound (IL-1 signalling inhibitor or senolytic) according to the invention may be used in a concentration between 0.01 µM and 20 µM, particularly, the compound of the invention may be used in a concentration of 0.01, 0.05, 0.1, 0.5, 1.0, 2.5, 5.0, 10.0, 15.0, 20.0 µM.

**In the second aspect,** the invention pertains to a compound for use in treating inflammatory side effects of irradiation therapy in a subject suffering from a solid tumour disease, wherein the compound is a senolytic or IL-1/IL-1R inhibitor.

The term "inflammatory side effects of irradiation therapy" or "inflammatory side effects of radiotherapy" as used herein shall pertain to a tumour phenotype characterized by the presence of inflammatory polarized CAF, and therefore, by the presence of an inflammatory gene signature (such as a signature shown in the enclosed examples). Such inflammatory side effects may be observed if one or more of the inflammatory markers are observed in the tumour such as selected from Cxcl1, Cxcl2, Cxcl5, Mmp9, Mmp13, Ilia, 116, Tnfa and Nos2.

In this specific second aspect the compound is administered to the subject in an amount effective to inhibit irradiation induced cell senescence in CAFs of the tumour. Effective amounts are described herein elsewhere. Preferably the compound for use of the invention when administered to the subject in said therapeutically effective amount has no irradiation-independent anti-tumour effect.

In **the third aspect**, the method for identifying or characterizing a compound suitable for increasing sensitivity of a solid tumour disease to irradiation therapy, the method comprising the steps of
- Contacting a candidate compound and a cancer-associated fibroblast (CAF),
- Inducing a p53 senescence program in the CAF contacted with the candidate compound,
wherein a reduced pro-inflammatory phenotype in the a contacted with the candidate compound compared to a CAF not contacted with the candidate compound indicates the candidate compound suitable for increasing sensitivity of a solid tumour disease to irradiation therapy.

Preferably step (b) involves induction of IL-1 signalling, for example using IL-1α, in the CAF or irradiation of the CAF.

In some embodiments, the pro-inflammatory phenotype involves increased gene expression of any one or any combination of: Cxcl1, Cxcl2, Cxcl5, Mmp9, Mmp13, Il1a, Il6, Tnfa and Nos2.

In **the fourth aspect**, the invention pertains to a method of treating a solid tumour disease in a subject in need thereof, the method comprising the steps of administering to the subject a therapeutically effective amount of a compound recited in any of the preceding claims, and irradiating the solid tumour in the subject.

Such treatment methods of the invention are performed in preferred embodiments in accordance with the medical uses described in the above first and second aspects, which preferred disclosures and definitions apply for this fourth aspects.

In context of the various aspects of the invention any compound may be formulated in a composition with either any carrier and/or excipient and/or additional therapeutic agents.

Preferred are pharmaceutical compositions, which comprise the compounds of the invention and a pharmaceutically acceptable carrier and/or excipient. "Pharmaceutically" or "pharmaceutically acceptable" refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Typically, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms. The compound of the present invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin. Sterile injectable solutions are prepared by incorporating the active compounds in the required amount in the appropriate solvent with several of the other ingredients enumerated above, as required, followed by filtered sterilization. Generally, dispersions are prepared by incorporating the various sterilized agent of the present inventions into a sterile vehicle which contains the basic dispersion medium and the required other ingredients from those enumerated above. In the case of sterile powders for the preparation of sterile injectable solutions, the typical methods of preparation are vacuum-drying and freeze-drying techniques which yield a powder of the compound of the present invention plus any additional desired ingredient from a previously sterile- filtered solution thereof. The preparation of more, or highly concentrated solutions for direct injection is also contemplated, where the use of DMSO as solvent is envisioned to result in extremely rapid penetration, delivering high concentrations of the active agents to a small tumor area. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. Some variation in dosage will necessarily occur depending on the condition of the patient being treated. The person responsible for administration will, in any event, determine the appropriate dose for the individual patient.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES

The figures show:

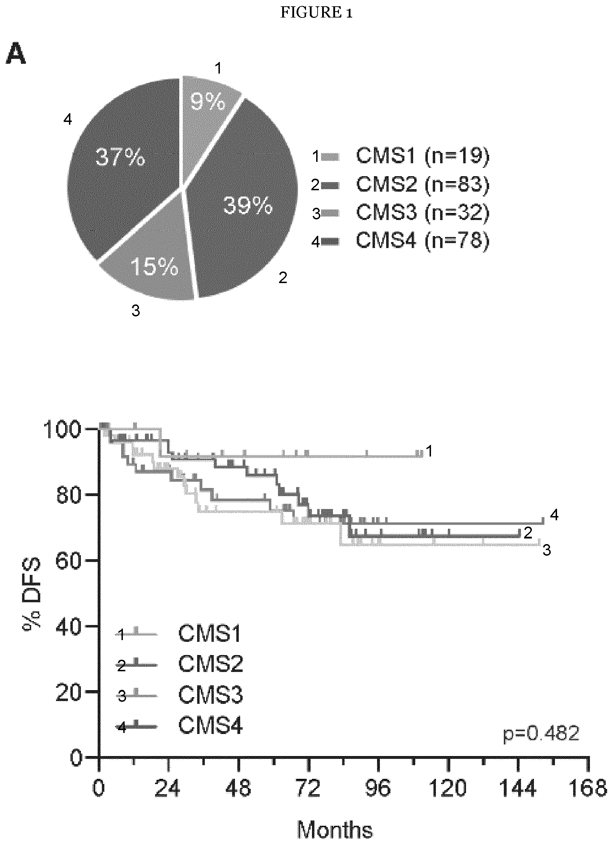

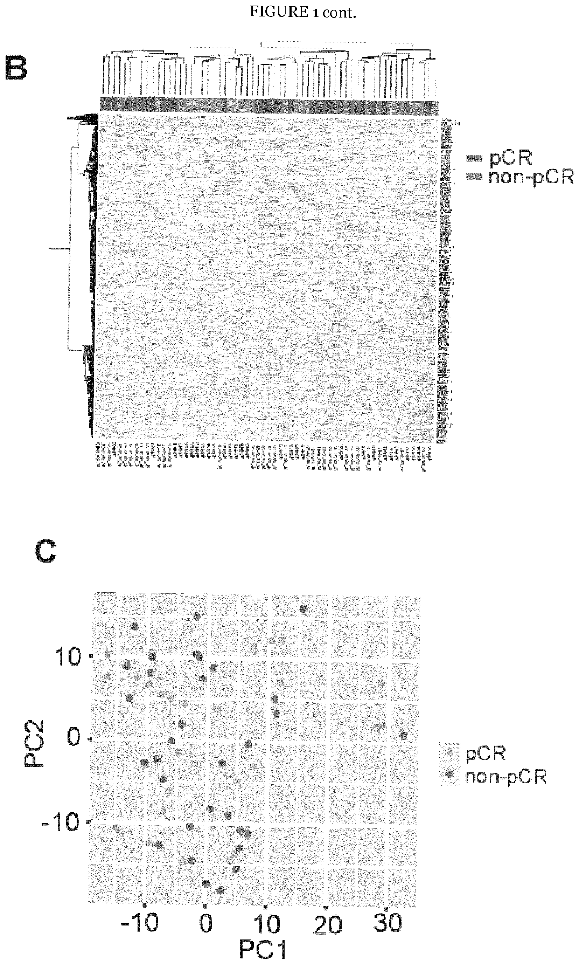

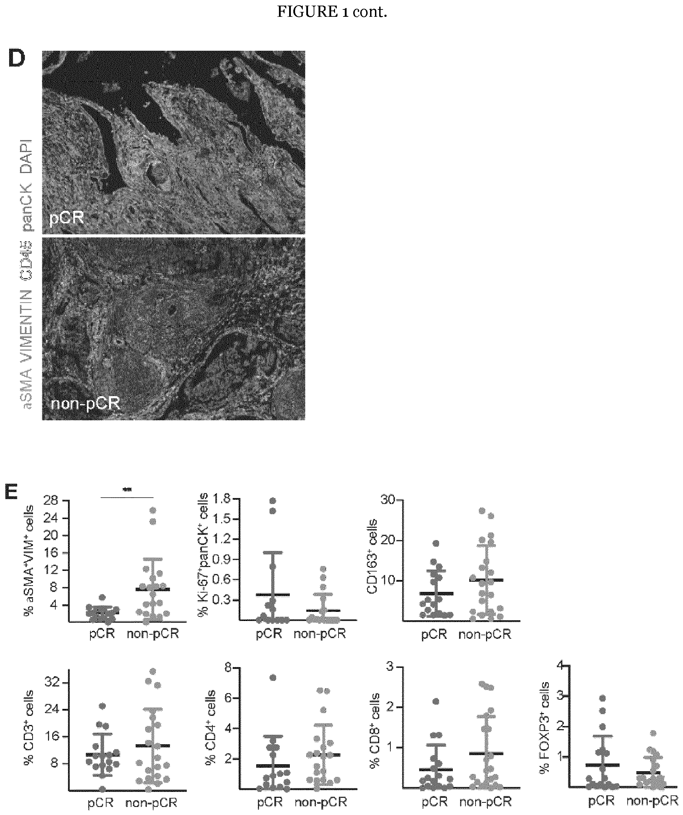

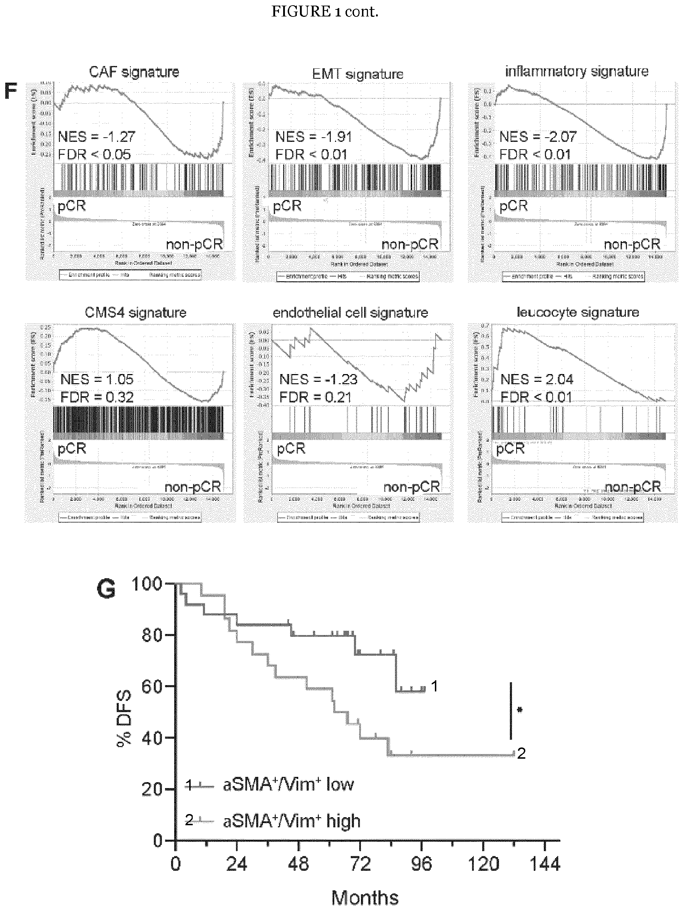

**Figure 1****:** shows that fibroblasts predict survival of rectal cancer patients. (A) Relative distribution and Kaplan-Meier-Survival curves of 212 rectal cancer patients according to their CMS classification. Note that only patients that were identified unanimously by at least two of the different algorithms were included into analysis. (B and C) Global proteomics analysis of laser capture microdissected tumor cells from pre-therapeutic biopsies of 29 pCR and 32 non-pCR patients using mass spectrometry: (B) heatmap; (C) PCA plot. (D and E) Representative multiplex immunofluorescent analysis of tumor biopsies from pCR and non-pCR patients before CRT; blue: DAPI; red: aSMA; white: CD45; green: Vimentin; magenta: panCK; scale bars = 200 µm; (E) quantification of: CD163, CD3, CD4, CD8, FOXP3, αSMA Vim and Ki67 panCK % of positive cells. Data are mean ± SD. N=16 pCR and 19 non pCR patients, ** p < 0.01 by t-test. (F) Gene set enrichment analysis (GSEA) for CAF, EMT, inflammatory, CMS4, endothelial cells and leukocytes signatures using pre-CRT RNA sequencing data of 105 patients' biopsies: 26 pCR & 79 non-pCR. Significance is for NES ≤ -1; FDR q-value <0.05. (G) Kaplan- Meier Disease Free Survival (DFS) curve of patients with high (n=23) and low (n=23) CAFs (αSMA+ Vim+ double positive cells) score based on the multiplexed immunohistochemical analysis of pre-CRT sections. Median split for high and low scores. * p < 0.05 by Log-rank (Mantel-Cox) test.

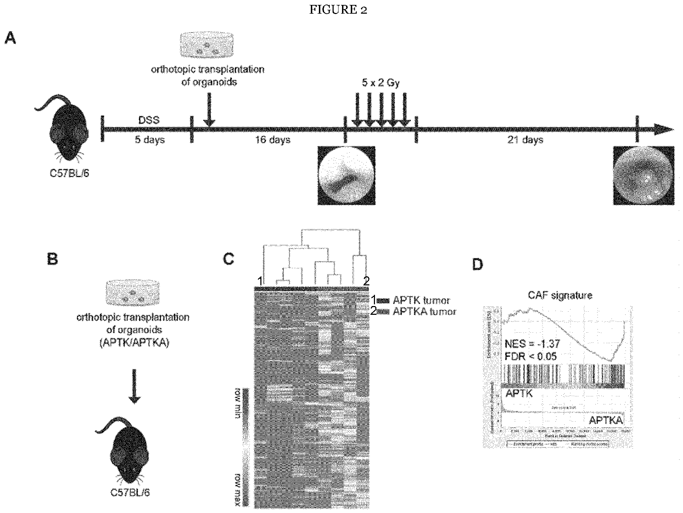

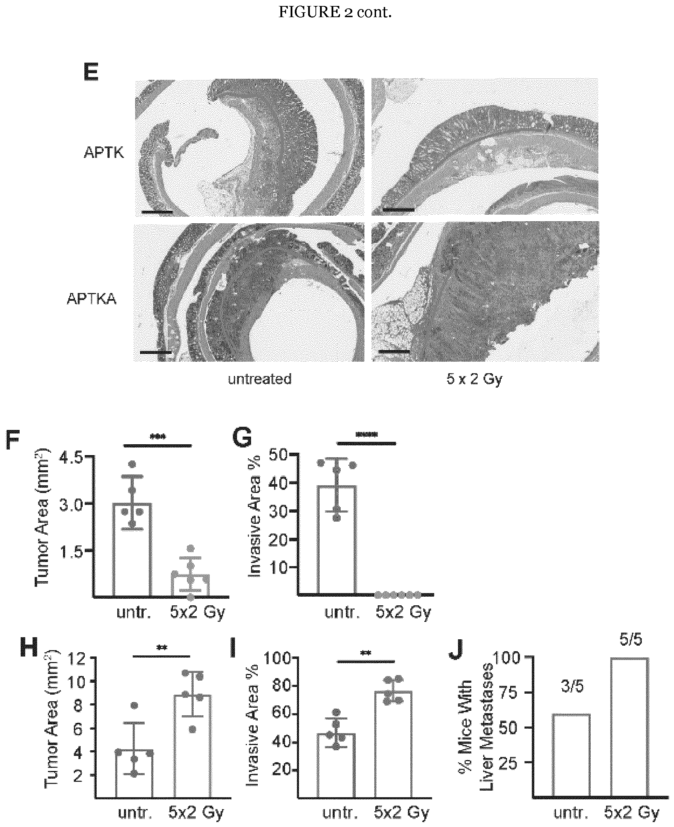

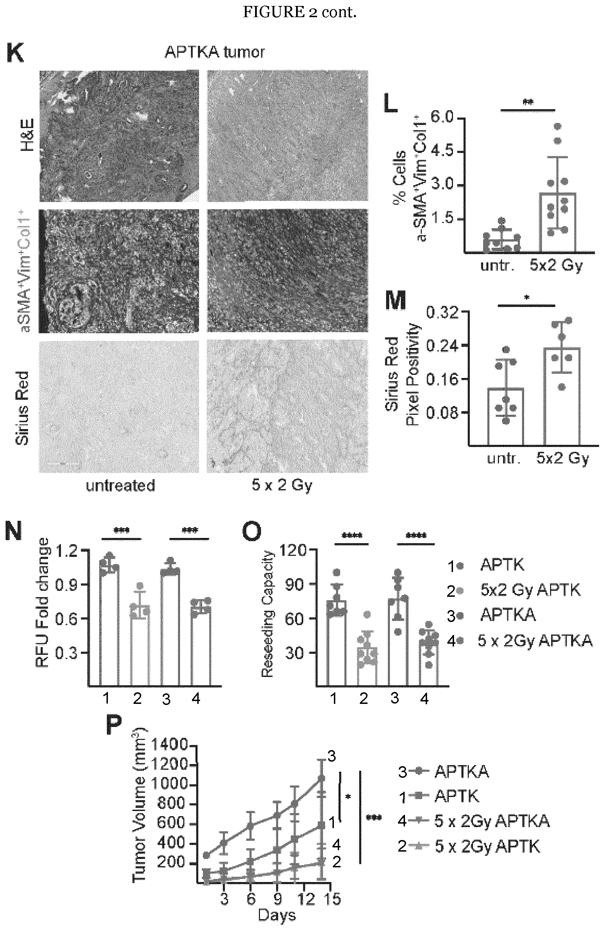

**Figure 2****:** shows orthotopic mouse model of rectal cancer. (A and B) Schematic representation of the orthotopic transplantation of APTKA & APTK mouse organoids into C57BL/6 mice followed by local fractionated radiotherapy (5 x 2 Gy) of established tumors and end point colonoscopy 21 days after the last irradiation. (C) Heat map analysis of RNA sequencing data of APTKA (n=4) and APTK (n=5) orthotopic tumors. (D) GSEA of CAF signature using RNA sequencing data of APTKA and APTK orthotopic tumors. Significant NES ≤-1; FDR q-value < 0.05. (E) H&E stained sections of APTK and APTKA untreated and irradiated orthotopic tumors 21 days after last irradiation. Scale bars = 500 µm. (F and G) Overall tumor (mm3) and invasive area (%) of untreated (n=5) and irradiated (n=6) APTK orthotopic tumors. Data are mean ± SD. *** p < 0.001 and **** p < 0.0001 by t-test. (H and I) Overall tumor (mm3) and invasive area (%) of untreated (n=5) and irradiated (n=5) APTKA orthotopic tumors. ** p <0.01 by t-test. Data are mean ± SD. (J) Frequency of mice with liver metastasis in untreated or irradiated APTKA orthotopic tumors (n=5 mice per group). (K) H&E staining of untreated and irradiated APTKA orthotopic tumors. Scale bars = 300 µm. Representative images of aSMA (Green), Vimentin (Blue) and Collagen (Yellow) multiplex immunofluorescence of untreated and irradiated APTKA tumors. Scale bars = 400 µm. Sirius red staining of APTKA orthotopic tumors upon radiotherapy. Scale bars = 300 µm. (L) Percent of αSMA, Vim and Col1 triple positive cells in untreated (n=9) and irradiated (n=10) APTKA tumors. Data are mean ± SD. ** p < 0.01 by t-test. (M) Sirius red pixel positivity in untreated (n=7) and irradiated (n=6) APTKA tumors. Data are mean ± SD. * p < 0.05 by t-test. (N-P) Ex vivo irradiation (5 x 2Gy) of APTKA and APTK organoids: (N) relative fluorescent units of untreated and irradiated organoids, 6 h post last dose, using resazurin cell viability kit (n= 4 independent wells per condition); (O) quantification of the number of fully grown untreated and irradiated organoids 4 days' post first reseeding (n=7 to 9 independent wells per condition); (P) subcutaneous tumor growth in C57BL/6 mice transplanted with untreated or irradiated organoids. Data are mean ± SD. N= 6 mice per group; p < 0.05 by one-way ANOVA followed by Tukey's multiple comparisons test. Representative results from one experiment which has been independently repeated least 3 times.

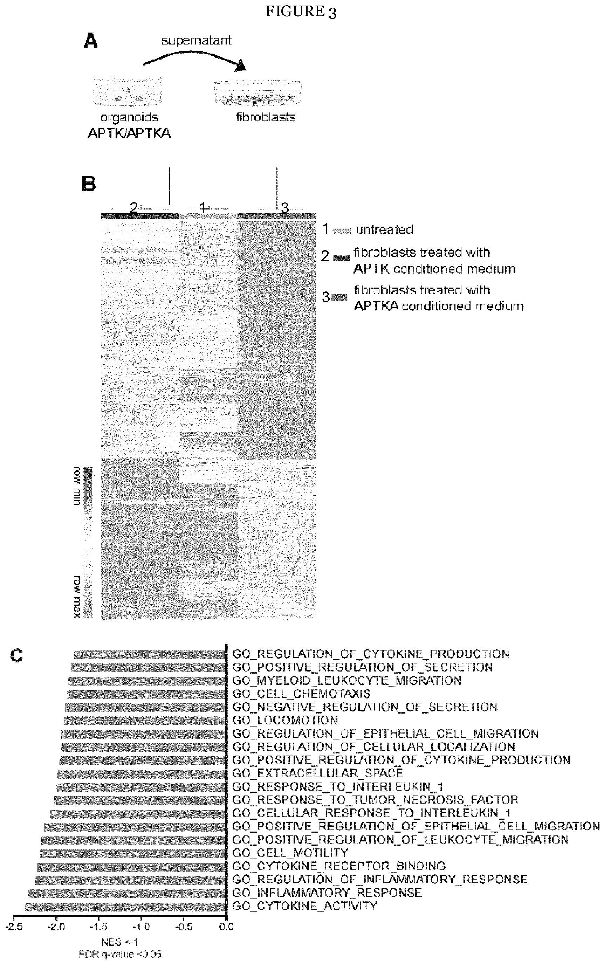

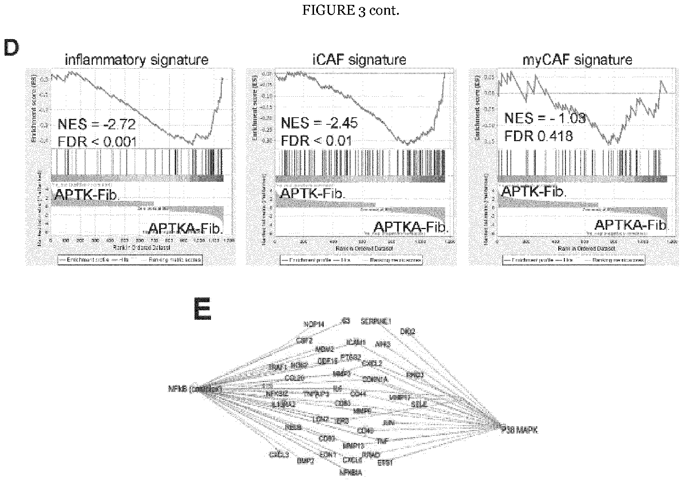

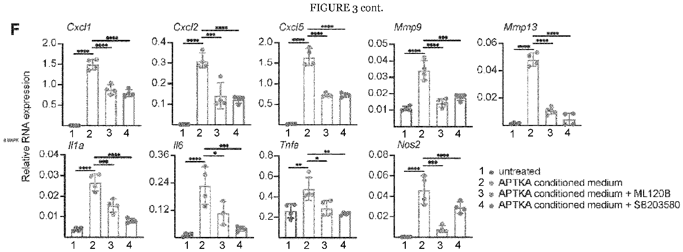

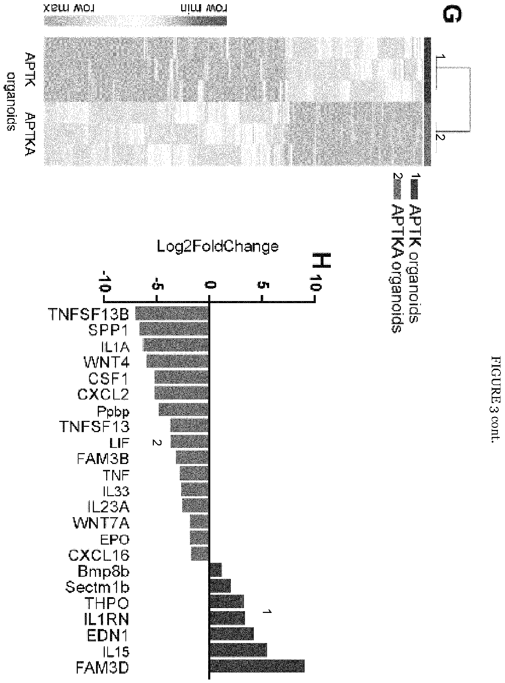

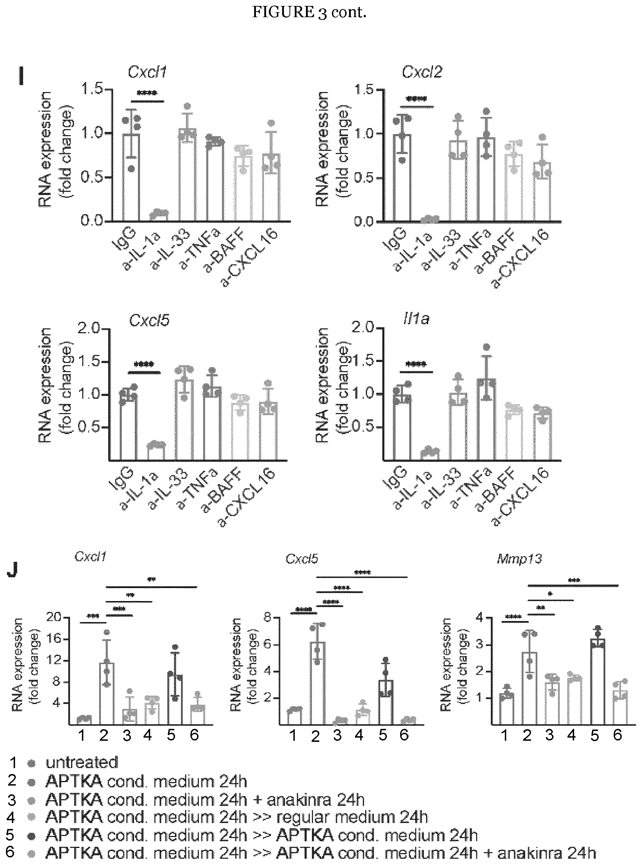

**Figure 3****:** shows that resistant tumors induce inflammatory CAF polarization in an IL-1α dependent manner. (A) Schematic illustration of APTKA or APTK organoids supernatant collection and treatment of primary mouse colon fibroblasts of unchallenged wildtype C57BL/6 mice. (B-E) RNA sequencing analysis of unchallenged (n=3), APTKA (n=4) or APTK supernatant (n=4) treated fibroblasts for 24 h: (B) Heat map of differential transcriptional profiles; (C and D) GSEA of Gene Ontology (GO) and CAF: inflammatory (iCAF) and myofibroblasts (myCAF) signatures, significantly enriched in APTKA compared to APTK media treated fibroblasts (15). NES ≤-1 and FDR q-Value <0.05; (E) Ingenuity- pathway interactive analysis of APTKA polarized fibroblasts transcriptomic profile suggesting NF-κB (activation z-score =-4.64, p-value <0.05) and p38 (activation z-score =-4.236, p <0.05) as upstream regulators of differentially expressed genes compared to APTK polarized fibroblasts. (F) Relative expression analysis by RT-qPCR of inflammatory genes in untreated wildtype and APTKA conditioned medium treated fibroblasts in the presence of IKKβ inhibitor ML120B (30µM) and p38 MAPK inhibitor SB203580 (10µM) for 24 h (n=4 independent wells per group). Data are mean ± SD. p < 0.05 by one-way ANOVA followed by Tukey's multiple comparisons test. Representative results from one experiment which has been independently repeated 3 times. (G and H) RNA sequencing analysis of APTKA (n=3) and APTK (n=3) organoids: (G) heat map analysis; (H) differentially expressed cytokines in APTKA (Log2 fold change ≤-1) compared to APTK organoids (log2 fold Change ≥1). p-value <0.05. (I) RT-qPCR analysis of inflammatory genes in APTKA conditioned fibroblasts treated with control IgG, α-IL-1α, α-IL-33, α-TNF-α, α-BAFF or α-CXCL16 neutralizing antibodies (2µg/mL each) for 24 h (n=4 independent wells per group). Data are mean ± SD. **** p < 0.0001 by one-way ANOVA followed by Tukey's multiple comparisons test. Experiment performed twice. (J) RNA expression profiles of inflammatory genes in APTKA conditioned fibroblasts stimulated for 24 h then subjected to anakinra (10µg/mL), APTKA fresh or regular medium treatments for another 24 h (n=4 independent wells per condition). Data are mean ± SD; p < 0.05 by one-way ANOVA followed by Tukey's multiple comparisons test. Representative results from one experiment which has been independently repeated 2 times.

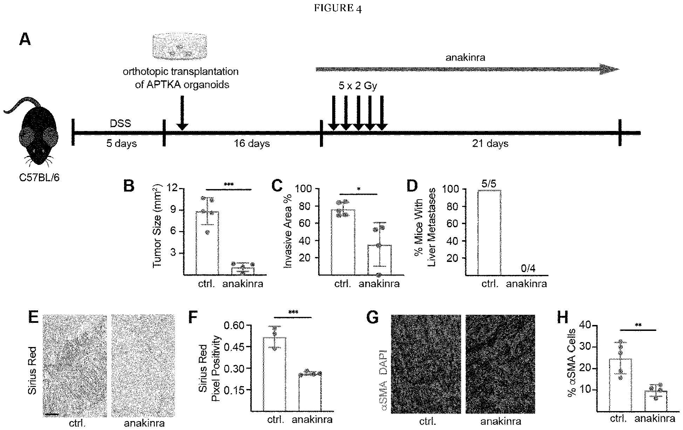

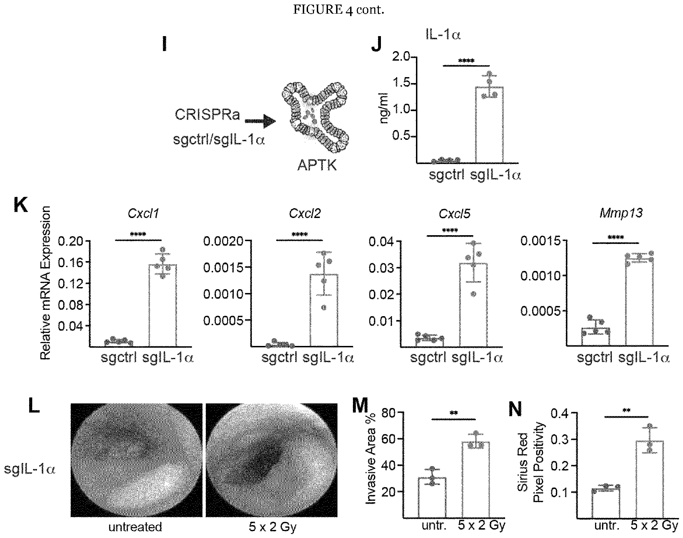

**Figure 4****:** shows that IL-1 is responsible for therapy resistance in orthotopic tumors. (A) Schematic representation of the orthotopic transplantation of APTKA mouse organoids into C57BL/6 mice followed by 5 x 2 Gy radiotherapy of established tumors untreated or treated daily with anakinra (500µg/day) for 21 days. (B and C) Overall tumor size (mm3) and % invasive area of 5 x 2Gy radiated APTKA orthotopic tumors 21 days after last irradiation without (n=5) or with (n=4) anakinra treatment (500µg/day). Data are mean ± SD; p <0.05 by t-test. (D) Frequency of mice with liver metastasis upon irradiation of APTKA orthotopic tumors without (n=5) or with (n=4) anakinra treatment (500µg/day). (E and F) Sirius red staining of untreated (n=3) or anakinra treated (n=4) 5 x 2 Gy irradiated APTKA tumors. Scale bars = 200µm. Data are mean ± SD. *** p < 0.001 by t-test. (G, H) aSMA immunofluorescent staining and its quantification of irradiated APTKA orthotopic tumors without (n=5) or with (n=4) anakinra treatment (500µg/day). Scale bars = 200 µm. Data are mean ± SD; p <0.05 by t-test. (I) Schematic representation of IL-ia overexpression in APTK organoids by CRISPRa/Cas9. (J) ELISA of IL-ia protein levels in conditioned media from APTK sgctrl and APTK sgIL-1α organoids (n= 4 independent wells per group). Data are mean ± SD. **** p < 0.0001 by t-test. (K) Relative RNA expression of inflammatory genes in fibroblasts treated with conditioned media from APTK sgctrl and APTK sgIL-1α organoids for 24 h (n=5 independent wells per condition). Data are mean ± SD. **** p < 0.0001 by t-test. Representative results from one experiment which has been independently repeated 2 times. (L) Colonoscopy of APTK sgIL-1α orthotopic tumor before (untreated) and 21 days after irradiation (5 x 2 Gy). (M) % of invasive area in untreated and 5 x 2 Gy irradiated APTK sgIL-1α orthotopic tumors (n= 3 mice per group). Data are mean ± SD. ** p < 0.01 by t-test. (N) Quantification of sirius red staining of untreated and irradiated APTK sgIL-1α orthotopic tumors (n= 3 mice per group). Data are mean ± SD. ** p-value = 0.01 by t-test.

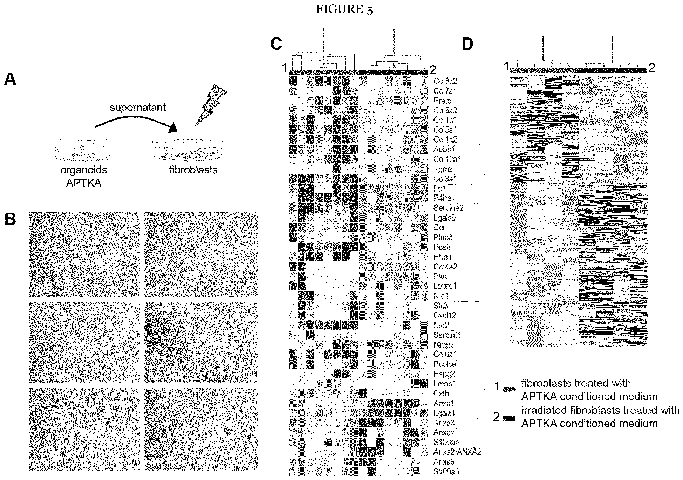

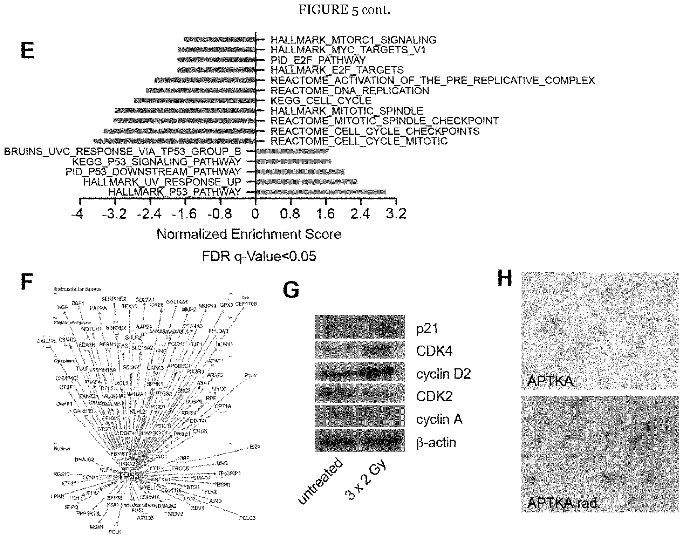

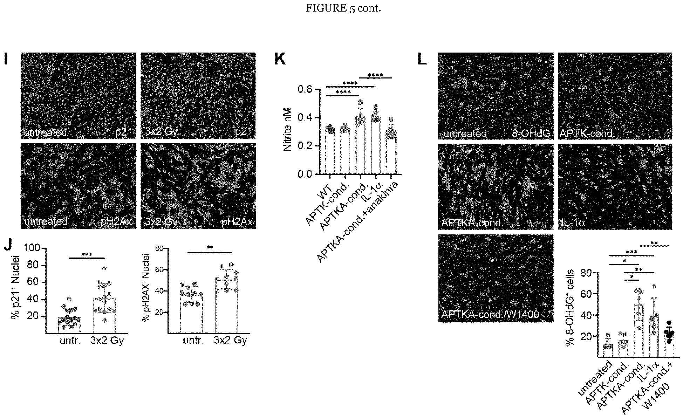

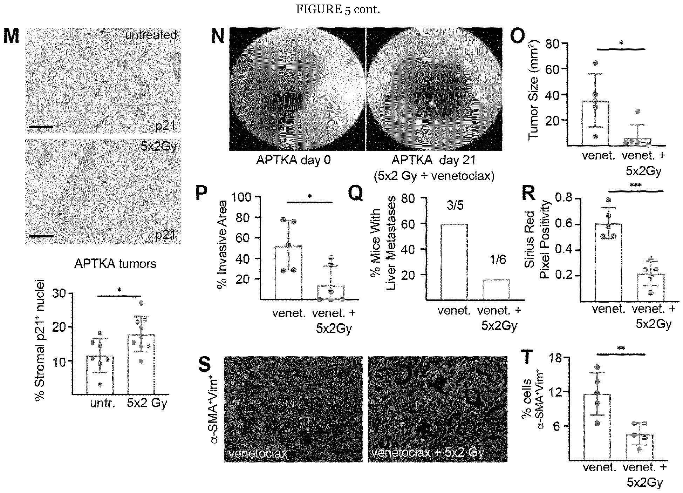

**Figure 5****:** shows that irradiation of inflammatory fibroblasts induces senescence. (A) Schematic illustration of APTKA organoids supernatant collection and treatment of colon fibroblasts from unchallenged wildtype C57BL/6 mice for 12 h followed by 3 x 2 Gy irradiation. Microscopic, proteomic & transcriptomic analysis were performed 72 h post first irradiation. (B) Microscopic captures of non-irradiated wildtype (WT) and APTKA conditioned fibroblasts. Remaining captures belong to irradiated fibroblasts: WT, IL-1α (1µg/mL) stimulated WT, and APTKA conditioned media minus or plus anakinra (20µg/mL) treated fibroblasts. Scale bars = 200µm. (C) Mass spectrometric analysis of matrisome profiles of 3 x 2 Gy irradiated (n=8) and non-irradiated (n=8) APTKA conditioned fibroblasts. Heat map represents significantly enriched (student' s t-test q-value <0.05) ECM related proteins. (D-F) RNA sequencing analysis of untreated (n=4) and irradiated (n=4) APTKA polarized fibroblasts: (D) heat map analysis; (E) GSEA in irradiated (NES ≥ 1, FDR q-Value <0.05) compared to untreated (NES ≤ - 1, FDR q-Value <0.05) APTKA polarized fibroblasts; (F) ingenuity- pathway interactive analysis of radiated APTKA fibroblasts transcriptomic profile suggesting TP53 (activation z-score = 5.882, p- value <0.05) as upstream regulator. (G) Immunoblot analysis of untreated and irradiated APTKA conditioned fibroblasts. Representative results from one experiment which has been independently repeated 2 times. (H) SA-β gal staining of untreated and irradiated APTKA fibroblasts. Scale bars = 100µm. (I and J) p21 and pH2AX immunofluorescent staining of untreated and irradiated APTKA fibroblasts. Scale bars = 200 µm. Data are mean ± SD. ** p < 0.001 and *** p < 0.001 by t-test. (K) Nitrite concentration determined by Griess assay in conditioned media from WT, APTK media, APTKA media, IL-1α (1µg/mL) stimulated and APTKA plus anakinra (20 µg/mL) treated fibroblasts for 72 h (n=10 independent wells per group). Data are mean ± SD. p-value <0.05 by one-way ANOVA followed by Tukey' s multiple comparisons test. Representative results from three independent experiments. (L) 8-OHDG immunofluorescent staining of WT, IL-1α (1µg/mL) stimulated, APTK media, APTKA media minus or plus NOS2 inhibitor W1400 (imM) treated fibroblasts for 72 h (n=5 independent wells per group). Scale bars = 200 µm. Data are mean ± SD. p < 0.05 by one-way ANOVA followed by Tukey's multiple comparisons test. (M) p21 immunohistochemistry of untreated (n=7) and irradiated (n=9) APTKA orthotopic tumors 21 days' post last dose RT. Scale bars represent 200 µm. Data are mean ± SD. * p < 0.05 by t-test. (N) Colonoscopy of APTKA orthotopic tumor before therapy and 21 days' post 5 x 2 Gy irradiation and daily venetoclax treatment (100mg/kg/day). (O and P) Overall tumor size and % of invasive area of venetoclax treated non irradiated (n=5) and irradiated (n=6) APTKA orthotopic tumors 21 days' post RT. Data are mean ± SD. P-value < 0.05 by t-test. (Q) Frequency of liver metastasis in venetoclax treated APTKA tumors without (n=5) or with (n=6) radiotherapy. (R) Sirius red staining of venetoclax treated APTKA tumors without (n=5) or with (n=5) 5 x 2 Gy radiotherapy. Data are mean ± SD. p <0.05 by t-test. (S and T) αSMA (red) Vimentin (green) immunofluorescent staining of venetoclax treated APTKA tumors without (n=5) or with (n=5) irradiation. Scale bars = 200µm. Data are mean ± SD. **p <0.001 by t-test.

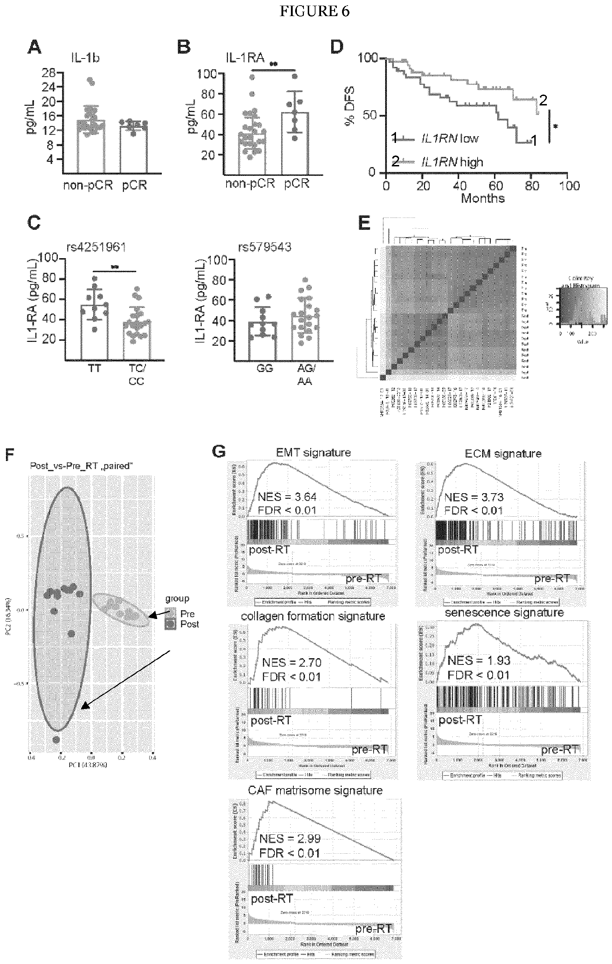

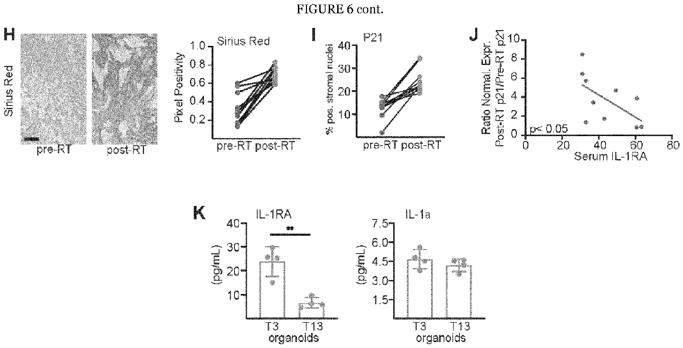

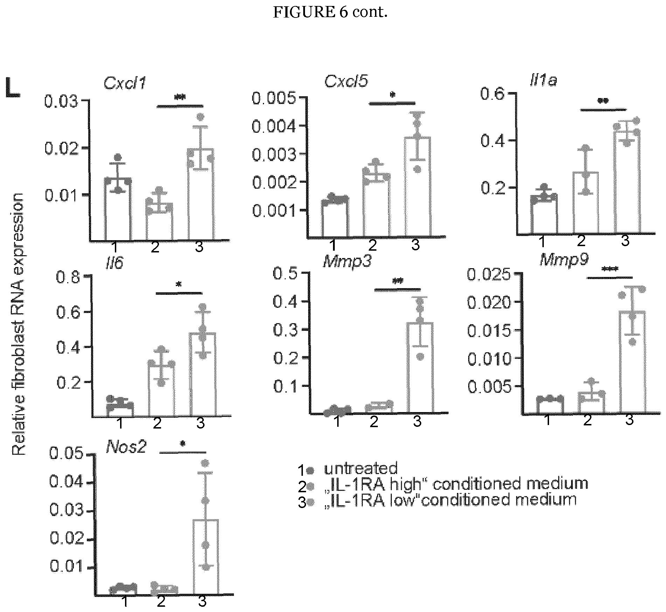

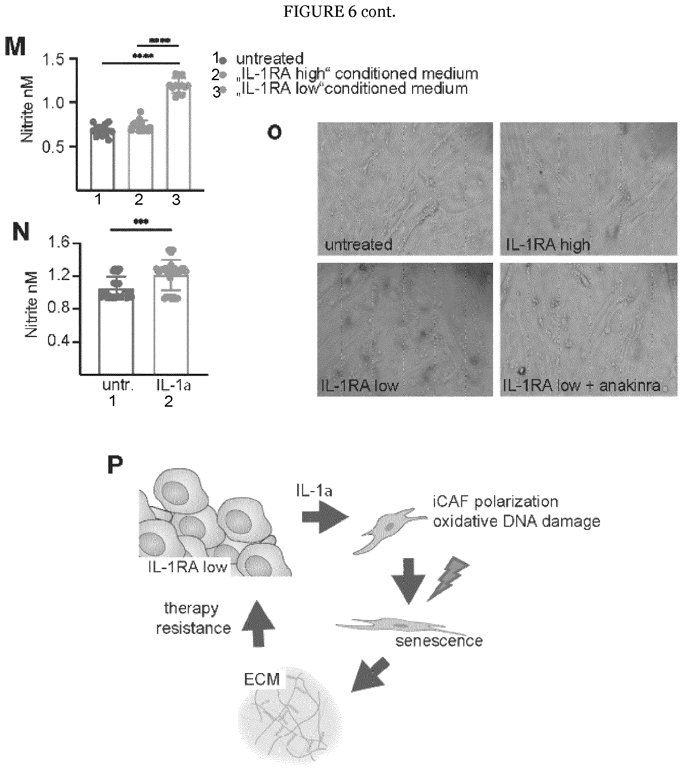

**Figure 6****:** shows that low IL-1RA levels enhance IL-1 signaling in rectal cancer patients and sensitize CAFs to therapy-induced senescence. (A and B) Quantification of IL-1β and IL-1RA baseline serum levels in non-pCR (n=25) and pCR (n=7) patients before CRT. Data are mean ± SD. ** p < 0.01 by t-test. (C) IL-1RA serum level association with two SNPs: rS4251961 (T/C) and rs579543 (A/G) using DNA isolated from patients (n=31) pre-CRT peripheral blood mononuclear cells. Data are mean ± SD. ** p =0.0052 by t-test. (D) Kaplan-Meier DFS curve of patients with high (n=37) and low (n=37) IL-1RN expression score (median split) based on the RNA sequencing data of pre-CRT biopsies. * p < 0.05 by Log-rank (Mantel-Cox) test. (E and G) Paired analysis of non-pCR patients (n=12) post-CRT versus pre-CRT RNA sequencing data: (E) Heat map; (F) PCA Plot and (G) GSEA of EMT, ECM, collagen formation, senescence & CAF matrisome signatures, significantly enriched post-therapy (NES≥1 & FDR q-value<0.05). (H) Paired analysis of sirius red staining of non-pCR patients (n=11) pre versus post-CRT. Scale bars = 300 µm. (I) Paired quantification of p21 immunohistochemistry staining of non-pCR patients (n=10) pre versus post CRT. (J) Correlation analysis of pre-CRT serum IL-1RA levels with ratio of p21 induction (post/pre-CRT p21 RNA expression level) (n=10 non pCR patients). * p < 0.05 by Spearman r correlation test. (K) IL-1RA and IL-ia protein levels in conditioned media from T3 and T13 human rectal cancer organoids (n=4 independent wells per group). Data are mean ± SD. ** p-value < 0.05 by t-test. (L) RT-qPCR analysis of inflammatory genes in untreated, IL-1RA high & IL-1RA low conditioned media treated human fibroblasts (n=4 independent wells per group). Data are mean ± SD. Significant p < 0.05 by one-way ANOVA followed by Tukey's multiple comparisons test. Representative results from one experiment which has been independently repeated at 2 times. (M) Nitrite concentration determined by Griess assay in supernatants from WT, IL-1RA high & IL-1RA low conditioned media treated human fibroblasts for 72 h (n=13 independent wells per group). Data are mean ± SD. Significant p-value <0.05 by one-way ANOVA followed by Tukey's multiple comparisons test. (N) Nitrite concentration determined by Griess assay in supernatants from untreated and IL-1α (1µg/mL) stimulated human fibroblasts for 72 h (n=32 independent wells per group representing 3 independent experiments). Data are mean ± SD. Significant p-value <0.05 by t-test. (O) SA-β gal staining of 3 x 2 Gy irradiated: WT, IL-1Ra high, IL1-Ra low with or without anakinra (20µg/mL) conditioned media treated human fibroblasts for 72 h. Scale bars = 100µm. (P) Summarizing scheme: In patients with low circulating IL-1RA levels, tumor cell derived IL-1α induces iCAF polarization and upregulation of iNOS, which elevates nitrite production in CAFs resulting in oxidative DNA damage. Upon irradiation DNA damage is further enhanced and triggers CAF senescence. Senescent CAFs change their secretory profile and produce more ECM, which counteracts irradiation-induced cell death and tumors, thus promoting tumor growth and inducing therapy resistance.

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Investigation of the role of fibroblasts for CRT response in distinct patient cohorts in comparison to other cells and biomarkers

The recently established consensus molecular subtype (CMS) classification for colon cancer based on transcriptomic profiles of whole tumors demonstrated that tumor subtyping allows prognosis prediction to some extent (8, 9).

Yet, when survival of 212 rectal cancer patients was compared, treated with neoadjuvant CRT and surgery and grouped according to three CMS classifier algorithms (CMSclassifier-random forest prediction, CMSclassifier-single sample prediction and CMScaller), a comparable outcome for the different subgroups (Fig.1A) was noticed indicating that subtyping of rectal cancer patients according to the CMS criteria does not allow DFS prediction (8, 9).

To identify possible predictive markers and signaling pathways that may be responsible for therapy response, a comprehensive global proteomic analysis of laser capture microdissected tumor cells derived from pre-therapeutic biopsies of 29 pCR (i.e. excellent prognosis) was performed after CRT and 32 patients with non-pCR (i.e. residual lymph node metastases with poor prognosis) after CRT using mass spectrometry. A total of 2747 proteins could be identified, however, principal component and heat map analysis of protein expression profiles did not reveal a distinct separation of the two patient cohorts (Fig. 1B, C) suggesting that possibly cells in the tumor microenvironment rather than the actual tumor cells may determine CRT response.

Indeed, a detailed multiplex fluorescent immunohistochemical analysis of pre-therapeutic biopsy samples of patients that either showed pCR or not revealed a significant enrichment of alpha-smooth muscle actin α-SMA+/Vimentin+ fibroblasts in pre-therapeutic biopsies from non-pCR patients (Fig.1D, E), while there was no difference in the number of T cells (CD3+, CD4+, CD8+ or Foxp3), macrophages (CD163) or Ki-67+ epithelial cells (panCK) (Fig. 1D, E). Moreover, an enrichment of a cancer-associated fibroblast (CAF) signature as well as an EMT and an inflammatory signature in transcriptomes of pretreatment biopsies from non-pCR patients (Fig. 1F) could be identified.

In contrast, there was no enrichment for an endothelial signature or a leukocyte signature in non-pCR patients at baseline further highlighting the relevance of fibroblasts for an impaired therapy response of these patients (10). Importantly, patients with high tumor expression of alpha-smooth muscle actin α-SMA+/Vimentin+ fibroblasts in the pre-therapeutic biopsies had a significantly worse DFS compared to patients with low expression (Fig. 1G). Surprisingly, yet in line with the lack of difference in DFS (Fig.1A), no enrichment in a mesenchymal CMS4 signature was also found in the two patient cohorts (11). However, the presence of an enriched inflammatory signature suggested that a distinct CAF polarization profile may confer resistance to CRT (12).

### Example 2: Confirmation of importance of inflammatory CAFs by a novel preclinical model of rectal cancer

To functionally confirm the importance of inflammatory CAFs for rectal cancer therapy and to dissect the underlying mechanism, a novel preclinical model of rectal cancer that allows monitoring of the effects of local irradiation in vivo was established. In this model tumor organoids are orthotopically transplanted into the distal lumen of C57BL/6 mice. Once growth of single tumors is confirmed by mini-endoscopy, mice undergo an image-guided radiation therapy using a small animal radiation research platform (SARRP) (Fig. 2A and Ext. Fig.1).

The inventors could recently demonstrate that orthotopic transplantation of organoids mutant for Apc, Trp53, Tgfbr2 and K-rasG12D (APTK) induce single invasive tumors in the distal colorectum that spontaneously metastasize to the liver in about 10% (13). Yet, transplantation of APTK organoids that in addition express myristoylated AKT (APTKA) develop tumors that are characterized by a more pronounced stromal response associated with a more aggressive phenotype and a higher frequency of spontaneous liver metastases (∼ 60%) (13). Remarkably, APTKA-tumors display an enrichment of CAF-associated genes (Fig. 2B-D) resembling the enhanced stroma reaction seen in the pre-therapeutic biopsies of patients with non-pCR after CRT.

The response of either APTK or APTKA-derived rectal tumors to a fractionated irradiation schedule (5 x 2 Gy) was compared in vivo, APTK-derived tumors responded well to radiation therapy (Fig. 2E) and showed a massive reduction in tumor size 21 days after therapy initiation (Fig. 2F, G). In stark contrast, APTKA-induced tumors were completely resistant to local irradiation (Fig. 2E) and displayed even more invasion and a higher frequency of liver metastases after irradiation when compared to untreated APTKA tumors (Fig. 2H-J). Interestingly, this was paralleled by a further increase of α-SMA+/vimentin+/collagen-1+(Col-1) fibroblasts and a change in fibroblast morphology that appeared in a more aligned pattern (Fig. 2K, L), which is usually associated with a change in extracellular matrix constituents and enhanced tumor progression (14). Increased collagen content was confirmed by sirius red staining (Fig. 2K, M). Importantly, however, APTK and APTKA organoids were equally sensitive to 5 x 2 Gy irradiation when treated ex vivo (Fig. 2N, O) and tumor growth was markedly retarded in both cases when organoids were injected subcutaneously following ex vivo irradiation (Fig. 2P) strongly supporting the notion that APTKA organoids were not intrinsically resistant to irradiation, but that this was rather mediated by stromal cells when tumor organoids were grown and treated in vivo.

To examine whether APTK and APTKA tumor organoids differentially affect surrounding fibroblasts in tumors directly in a paracrine manner, supernatants were collected from both tumor organoid lines and treated primary intestinal fibroblasts of unchallenged wildtype mice for 24 hours with the two different tumor-conditioned media ex vivo (Fig. 3A). RNAseq analysis confirmed distinct gene expression patterns in fibroblasts exposed to the two organoid supernatants (Fig. 3B). Gene set enrichment analysis (GSEA) revealed a strong inflammatory polarization in fibroblasts exposed to APTKA conditioned supernatants (Fig. 3C, D) and a distinct enrichment of inflammatory CAF (iCAF) but not myofibroblasts (myCAF)-associated genes (Fig. 3D), thus resembling the phenotype which was found in whole tumors in vivo (15).

Ingenuity-pathway analysis (IPA) suggested NF-κB and p38 activation as the main signaling pathways responsible for the inflammatory gene expression profile (Fig. 3E). Indeed, treating wildtype fibroblasts with APTKA conditioned medium in the presence of the specific IKKβ inhibitors ML120B or the p38 inhibitor SB203580 significantly inhibited the induction of pro-inflammatory genes such as Cxcl1, Cxcl2, Cxcl5, Mmp9, Mmp13, Il1a, Il6, Tnfa and Nos2 (Fig. 3F).

The inventors then aimed to identify the upstream factors secreted by APTKA organoids that were responsible for activating NF-κB and p38 in fibroblasts. To this end RNAseq analysis of APTK and APTKA tumor organoids were performed and identified several differentially expressed genes encoding cytokines (Fig. 3G, H). Using neutralizing antibodies, NF-κB and p38 inducing cytokines TNFα, IL-1α, IL-33, BAFF or CXCL16 were blocked in APTKA supernatants and expression of Cxcl1, Cxcl2, Cxcl5 and Il1a as inflammatory marker genes in fibroblasts was examined. In line with the previously described role of IL-1 in inflammatory CAF polarization in pancreatic cancer (16), only inhibition of IL-1α prevented upregulation of pro-inflammatory genes in fibroblasts challenged with conditioned medium from APTKA organoids (Fig. 3I), which could be confirmed using recombinant IL-ra (anakinra) or Il-1r-/- fibroblasts (data not shown). Conversely, recombinant IL-1α alone sufficed to induce a similar transcriptomic profile as APTKA supernatant in fibroblasts (data not shown).

It was then examined whether polarization towards an inflammatory CAF represented a stable state or whether blocking IL-1α does not only prevent but may also allow reversion of the established inflammatory phenotype as this would have implications for the therapy of tumors with a distinct CAF profile. To do so, fibroblasts were stimulated for 24 hours with APTKA conditioned medium before adding anakinra, adding fresh APTKA medium or changing medium to regular medium. Interestingly, simply replacing APTKA medium with regular medium reduced gene expression of Cxcl1, Cxcl5 and Mmp13 in fibroblasts (Fig. 3J). Moreover, also addition of anakinra to APTKA medium completely reverted expression of these genes indicating that organoid-derived IL-1α was required to maintain CAF polarization.

### Example 3: Investigation of improving response to radiation therapy by IL-1α blockade

To examine whether IL-ia blockade would improve the response to radiation therapy of APTKA tumors in vivo, mice were treated with anakinra (500µg/day) once tumors had established (Fig. 4A). While anakinra administration alone did not affect APTKA tumor growth significantly (data not shown), it led to a marked reduction in tumor size and invasion (Fig. 4B, C) and blocked metastasis formation when APTKA tumors were irradiated (Fig. 4D). This was accompanied by a significant decrease in sirius red staining (Fig. 4E, F) as well as a decreased number of □SMA+ cells (Fig. 4G, H) indicating that anakinra had a pronounced impact on CAF activation and suggested that tumor cell derived IL-1□ represented a key factor in the development of therapy resistance of murine rectal tumors. To further confirm this, Il1α transcription was activated in irradiation-sensitive APTK organoids by CRISPRa/Cas9 (Fig. 4I, J). When fibroblasts were exposed to conditioned medium of APTK-sgIL-1α organoids they started expressing an inflammatory gene expression profile that was comparable to the APTKA-induced one (Fig. 4K). Moreover, increased IL-ia release by APTK organoids rendered the corresponding tumors resistant to radiation therapy in the orthotopic in vivo model (Fig. 4L). APTK-sgIL-1α tumors did not decrease in size but became more invasive (Fig. 4M). Similar to APTKA-tumors, APTK-sgIL-1α tumors displayed an enhanced stroma reaction upon irradiation, characterized by an increased sirius red staining (Fig. 4N). Collectively, these data supported the conclusion that tumor cell-derived IL-ia polarizes CAFs towards an inflammatory phenotype, which then causes resistance to irradiation.

To explore how CAFs would confer therapy resistance in our model, it was examined whether exposure of fibroblasts to conditioned medium of irradiated tumor organoids would change the CAF phenotype (Ext. Fig. S2A). However, RNAseq analysis did not reveal any marked changes in gene expression when fibroblasts were compared that were subjected to medium derived from irradiated or non-irradiated organoids (Ext Fig. S2B). Therefore, it was tested whether APTK and APTKA-polarized fibroblasts themselves may be affected differently by irradiation. Primary fibroblasts were treated with APTK or APTKA conditioned medium or left them untreated and then subjected them to a fractionated irradiation (3 x 2Gy; Fig. 5A). This led to marked morphological differences between the differently treated fibroblasts and when compared to unchallenged or APTK-polarized fibroblasts, APTKA-educated fibroblasts developed a more elongated, spindle like shape (Fig. 5B) that clearly resembled the phenotype observed in vivo (Fig. 2K). Administration of anakinra to wildtype fibroblasts or use of Il1-r-/- fibroblasts prevented the APTKA-induced phenotype, while single application of recombinant IL-1α was sufficient to cause a comparable morphology after irradiation (Fig. 5B and data not shown), thus, clearly demonstrating the IL-1α-dependent morphological change. Elongation of fibroblasts is associated with changes in ECM constituents (14). Therefore, supernatants of irradiated and non-irradiated APTKA-polarized fibroblasts were collected and the ECM components (matrisome) were characterized by mass spectrometry (17, 18). In line with the morphological changes, irradiation led to a significant increase in various ECM glycoproteins, collagens and proteoglycans as well as ECM-regulators and growth factors (Fig. 5C) many of which are known to confer substantial support for tumor progression (19, 20).

Furthermore, transcriptomes of irradiated and non-irradiated APTKA-educated fibroblasts were compared by RNAseq analysis, which revealed marked changes and a downregulation of cell cycle and cell cycle checkpoint associated genes as well as induction of genes controlling senescence (Fig. 5D, E). Moreover, IPA indicated that many of the induced genes including those encoding various secreted factors (extracellular proteins: collagens, proteoglycans, growth factors, ECM regulators, ECM affiliated proteins) that also had been identified in the matrisome analysis as well as the cell cycle inhibitor Cdkn1a were regulated in a p53-dependent manner (Fig. 5F) suggesting induction of a p53-dependent senescence in APTKA-polarized fibroblasts upon irradiation.

In agreement with this notion and the presence of a growth arrest of fibroblasts, immunoblot analysis confirmed upregulation of p21, cdk4, cyclin D2 and downregulation of cdk2 and cyclin A (Fig. 5G), while SA-βgal staining validated senescence in irradiated fibroblasts that had been exposed to APTKA conditioned medium before (Fig. 5H). Senescence induction was dependent on IL-1 (Ext. Fig. 2C) and was accompanied by markedly enhanced DNA damage (Fig. 5I). To understand how IL-ia would sensitize fibroblasts to irradiation-induced senescence, the inventors hypothesized that IL-ia induced formation of reactive oxygen and nitrogen species (ROS and RNS), which in turn triggered oxidative DNA damage. Subsequent irradiation would enhance DNA damage further and initiate a p53-dependent senescence program.

However, it was not able to detect a higher level of ROS in fibroblasts exposed to APTKA-conditioned medium (data not shown). In line with meeting the altered energetic demand, due to decreased proliferation and increased quiescence, mitochondrial function in APTKA-conditioned fibroblasts was declined. Cells displayed lower oxygen consumption and extracellular acidification rate as well as lowered maximal respiration and reserve respiratory capacity (Ext. Fig.2F). Interestingly, also basal glycolysis as well as compensatory glycolysis, when mitochondrial respiration was blocked, remained lower in these cells (Ext. Fig. 2G). In contrast, 72 hours after exposure to APTKA-conditioned medium or to recombinant IL-ia and in line with enhanced Nos2 expression (Fig. 3F) nitrite levels in fibroblast supernatants were significantly elevated (Fig. 5K) which was paralleled by an increase in oxidative DNA damage determined by 8-OHdG immunofluorescence (Fig. 5L). Administration of the Nos2 inhibitor W1400 blocked nitrite production and prevented oxidative DNA damage as well as senescence induction in fibroblasts that had been treated with APTKA-conditioned medium (Fig. 5L and Ext. Fig. 2C-E). In agreement with these findings, a significant increase in nuclear p21 in stroma cells of APTKA orthotopic tumors upon irradiation was detected (Fig. 5M) indicating that a senescence program was initiated also in vivo.

The inventors then aimed to confirm the functional relevance of CAF senescence for therapy resistance of APTKA-tumors and treated tumor bearing mice with the senolytic compound venetoclax (100mg/kg/day) in addition to RT. Indeed, targeting senescent cells sensitized APTKA-tumors to irradiation and led to a significant decrease in tumor load (Fig. 5N, O) and suppressed invasion and formation of liver metastases (Fig. 5P, Q) which was accompanied by a decrease in sirius red staining and α-SMA+/vimentin+ cell number (Fig. 5R-T). Collectively, these results confirmed that IL-1α-dependent polarization of inflammatory CAFs elevated nitrite production and oxidative DNA damage in these cells. Subsequent irradiation enhanced DNA damage further and triggered a p53-dependent senescence program including the secretion of cytokines and ECM constituents that support tumor invasion and metastasis of cancer cells to counteract the irradiation-induced tumor cell death.

Finally, the inventors aimed to examine whether the observed IL-1α-induced changes in the tumor microenvironment of our murine model would be associated with poor therapy response in rectal cancer patients. To this end, serum samples were analyzed using Luminex Bio-Plex assay and determined serum levels of IL-1α, IL-1β, IL-1RA as well as IL-2, IL-4, IL-5, IL-6, IL-7, IL-8, IL-9, IL-10, IL-12, IL-13, IL-15, IL-17, CCL2, CCL3, CCL4, CCL5, TNFα, FGF, PDGF, GM-CSF and Eotaxin in non-pCR patients (n=25) and compared them to patients that showed a pCR (n=7) after CRT. While IL-ia levels were below the detection limit in all patients examined, it could not be observed a significant difference for IL-1β or for any of 25 other cytokines (Fig. 6A, Ext. Fig. 3). However, serum IL-1RA levels were markedly reduced in non-pCR patients with residual lymph node metastases and poor prognosis after CRT (Fig. 6B), suggesting that these patients indeed were characterized by an enhanced IL-1 signaling due to lower expression of the antagonist. IL-RA levels in vivo have been reported to be frequently associated with two single nucleotide polymorphisms (SNPs) in IL-1RA (rs4251961 T/C and rs579543 C/T) with higher IL-RA levels being observed in carriers of the homozygous rS4251961 T/T and rs579543 G/G genotypes (21).

An elevated IL-1RA serum levels in homozygous rS4251961 T/T patients could be confirmed, while no correlation between IL-1RA serum levels and the different rs579543 genotypes (Fig. 6C) was observed. Moreover, all pCR patients analyzed were carriers of the protective rS4251961 T/T or T/C alleles and none of them was a homozygous C/C carrier. Importantly, lower baseline IL1RN gene expression in pre-therapeutic tumor biopsies was associated with a significantly worse DFS (Fig. 6D) supporting the relevance of enhanced IL-1 signaling in therapy response. Then RNA expression was compared before and after CRT in 12 non-pCR patients (Fig. 6E). Principle component analysis revealed that CRT led to a clear clustering of RNA expression profiles (Fig. 6F). Furthermore, CRT induced an EMT, ECM and collagen formation signature in these patients (Fig. 6G). When a cross-species comparison was performed non-pCR patients showed an enrichment of a senescence signature that was established in irradiated APTKA-conditioned fibroblasts as well as a clear expression of the genes identified in the murine CAF matrisome (Fig. 6G). CRT induced a pronounced stromal response determined by sirius red staining (Fig. 6H) and in line with induction of stromal cell senescence, p21 expression increased in all patients in response to CRT (Fig. 6I), while the ratio of transcriptional p21 induction (post CRT/pre CRT) correlated inversely with the respective serum IL-1RA levels (Fig. 6J), thus strongly supporting a link between IL-1 signaling and therapy-induced stromal senescence in rectal cancer patients.

To further corroborate this, patient derived organoids (PDO) from biopsies taken before initiation of CRT from non-pCR patients were established and it was focused on organoids that were characterized by different IL-1RA but comparable IL-1α levels (Fig. 6K). Incubation of human intestinal fibroblasts with conditioned supernatants from "IL-1RA low" organoids induced a stronger pro-inflammatory gene expression profile than supernatants from "IL-1RA high" organoids (Fig. 6L) and led to higher nitrite production by fibroblasts (Fig. 6M), which could also be confirmed when fibroblasts were stimulated with IL-ia only (Fig. 6N). Importantly, SA-β-gal staining of fibroblasts demonstrated that this was associated with a stronger senescence induction in "IL-1RA low" pre-conditioned fibroblasts upon irradiation, which could be blocked by anakinra confirming the IL-1 dependence of therapy-induced senescence in CAFs (Fig. 6O).

### REFERENCES

The references are:
1. 1. F. R. Greten, S. I. Grivennikov, Inflammation and Cancer: Triggers, Mechanisms, and Consequences. Immunity 51, 27-41 (2019).
2. C. Rodel, R. Hofheinz, E. Fokas, Rectal cancer: Neoadjuvant chemoradiotherapy. Best Pract Res Clin Gastroenterol 30, 629-639 (2016).
3. R. Sauer et al., Preoperative versus postoperative chemoradiotherapy for locally advanced rectal cancer: results of the German CAO/ARO/AIO-94 randomized phase III trial after a median follow-up of 11 years. J Clin Oncol 30, 1926-1933 (2012).
4. W. van Gijn et al., Preoperative radiotherapy combined with total mesorectal excision for resectable rectal cancer: 12-year follow-up of the multicentre, randomised controlled TME trial. Lancet Oncol 12, 575-582 (2011).
5. E. Fokas et al., Outcome measures in multimodal rectal cancer trials. Lancet Oncol 21, e252-e264 (2020).
6. E. Fokas et al., Neoadjuvant rectal score as individual-level surrogate for disease-free survival in rectal cancer in the CAO/ARO/AIO-04 randomized phase III trial. Ann Oncol 29,1521-1527 (2018).
7. E. Fokas et al., Tumor Regression Grading After Preoperative Chemoradiotherapy as a Prognostic Factor and Individual-Level Surrogate for Disease-Free Survival in Rectal Cancer. J Natl Cancer Inst 109, (2017).
8. P. W. Eide, J. Bruun, R. A. Lothe, A. Sveen, CMScaller: an R package for consensus molecular subtyping of colorectal cancer pre-clinical models. Sci Rep 7, 16618 (2017).
9. J. Guinney et al., The consensus molecular subtypes of colorectal cancer. Nat Med 21, 1350-1356 (2015).
10. C. Isella et al., Stromal contribution to the colorectal cancer transcriptome. Nat Genet 47, 312-319 (2015).
11. B. E. Michels et al., Human colon organoids reveal distinct physiologic and oncogenic Wnt responses. J Exp Med 216, 704-720 (2019).
12. E. Sahai et al., A framework for advancing our understanding of cancer-associated fibroblasts. Nat Rev Cancer 20, 174-186 (2020).
13. J. Varga et al., AKT-dependent NOTCH3 activation drives tumor progression in a model of mesenchymal colorectal cancer. J Exp Med 217, (2020).
14. C. Gaggioli et al., Fibroblast-led collective invasion of carcinoma cells with differing roles for RhoGTPases in leading and following cells. Nat Cell Biol 9, 1392-1400 (2007).
15. D. Ohlund et al., Distinct populations of inflammatory fibroblasts and myofibroblasts in pancreatic cancer. J Exp Med 214, 579-596 (2017).
16. G. Biffi et al., IL1-Induced JAK/STAT Signaling Is Antagonized by TGFbeta to Shape CAF Heterogeneity in Pancreatic Ductal Adenocarcinoma. Cancer Discov 9, 282-301 (2019).
17. A. Naba, K. R. Clauser, R. O. Hynes, Enrichment of Extracellular Matrix Proteins from Tissues and Digestion into Peptides for Mass Spectrometry Analysis. J Vis Exp, e53057 (2015).
18. A. Naba et al., Characterization of the Extracellular Matrix of Normal and Diseased Tissues Using Proteomics. J Proteome Res 16, 3083-3091 (2017).
19. A. Naba et al., Extracellular matrix signatures of human primary metastatic colon cancers and their metastases to liver. BMC Cancer 14, 518 (2014).
20. C. Tian et al., Proteomic analyses of ECM during pancreatic ductal adenocarcinoma progression reveal different contributions by tumor and stromal cells. Proc Natl Acad Sci U S A 116, 19609-19618 (2019).
21. S. Rafiq et al., Common genetic variation in the gene encoding interleukin-i-receptor antagonist (IL-1RA) is associated with altered circulating IL-1RA levels. Genes Immun 8, 344-351 (2007).
22. K. Ganesh et al., A rectal cancer organoid platform to study individual responses to chemoradiation. Nat Med 25, 1607-1614 (2019).
23. Y. Yao et al., Patient-Derived Organoids Predict Chemoradiation Responses of Locally Advanced Rectal Cancer. Cell Stem Cell 26, 17-26 e16 (2020).
24. H. E. Barker, J. T. Paget, A. A. Khan, K. J. Harrington, The tumour microenvironment after radiotherapy: mechanisms of resistance and recurrence. Nat Rev Cancer 15, 409-425 (2015).

## Claims

1. **A compound for use in the treatment of a solid tumour** in a subject, wherein the subject is treated by reducing sensitivity of cancer-associated fibroblasts (CAFs) to cellular senescence and concomitant or subsequent irradiation therapy targeting the solid tumour in the subject.

2. The compound for use of claim 1, wherein the compound is a senolytic or an IL-1 signalling inhibitor.

3. The compound for use of claim 1 or 2, wherein the CAFs are located adjacent to, or within, the solid tumour in the subject.

4. The compound for use of any one of claims 1 to 3, wherein the compound increases tumour sensitivity of, or reduces tumour resistance to, irradiation therapy, preferably by inhibiting irradiation induced cell senescence in CAFs.

5. The compound for use of any one of claims 104, wherein the compound is an inhibitor/antagonist of inflammatory CAF polarization.

6. The compound for use of any one of claims 1 to 5, wherein the compound is an IL-1 inhibitor selected from rilonacept, anakinra, or biosimilars thereof, or an IL-1 or IL-1R antibody (preferably IL-1α/IL-1RA), such antibody may be selected from anti-IL-1R1 antibody AMG 108 (Amgen), chimeric, Canakinumab, humanized or human antibody directed to IL-1 alpha or beta (such as CDP-484, Celltech) or the IL-1 receptor (for example, AMG-108, Amgen; R-1599, Roche).

7. The compound for use of any one of claims 1 to 6, wherein the solid tumour is **characterized by** the presence of CAFs.

8. The compound for use of any one of claims 1 to 7, wherein the compound is a senolytic, selected from venetoclax, or Quercetin, Fisetin, Luteolin, Curcumin, A1331852, A1155463, Geldanamycin, Tanespimycin, Alvespimycin, Piperlongumine, FOXO4-related peptide, or Nutlin3a.

9. The compound for use of any one of claims 1 to 8, wherein the solid tumour is resistant to irradiation therapy.

10. The compound for use of any one of claims 1 to 9, wherein the subject has already received a first-line irradiation therapy, and has developed a resistance or reduced sensitivity to irradiation therapy.

11. **A compound for use in treating inflammatory side effects of irradiation therapy** in a subject suffering from a solid tumour disease, wherein the compound is a senolytic or IL-1/IL-1R inhibitor.

12. The compound for use of claim 11, wherein the compound is administered to the subject in an amount effective to inhibit irradiation induced cell senescence in CAFs of the tumour.

13. The compound for use of any one of the preceding claims, wherein the compound when administered to the subject in said therapeutically effective amount has no irradiation-independent anti-tumour effect.

14. **A method for identifying or characterizing a compound suitable for increasing sensitivity of a solid tumour disease to irradiation therapy,** the method comprising the steps of
(a) Contacting a candidate compound and a cancer-associated fibroblast (CAF),
(b) Inducing a p53 senescence program in the CAF contacted with the candidate compound,
wherein a reduced pro-inflammatory phenotype in the a contacted with the candidate compound compared to a CAF not contacted with the candidate compound indicates the candidate compound suitable for increasing sensitivity of a solid tumour disease to irradiation therapy.

15. The method of claim 14, wherein step (b) involves induction of IL-1 signalling, for example using IL-ia, in the CAF or irradiation of the CAF.
